# EUROPÄISCHE PATENTSCHRIFT

(11) **EP 0 662 815 B1**
(45) Veröffentlichungstag und Bekanntmachung des Hinweises auf die Patenterteilung: **15.01.1997**
(21) Anmeldenummer: 93914727.8
(22) Anmeldetag: 24.06.1993
(51) Int. Cl.: A61K 7/06, A61K 7/50

(54) **HAARBEHANDLUNGSMITTEL**
HAIR-CARE AGENT
AGENT DE SOINS CAPILLAIRES

(30) Priorität: 29.09.1992 DE 4232506
(43) Veröffentlichungstag der Anmeldung: 19.07.1995
(73) Patentinhaber: Henkel Kommanditgesellschaft auf Aktien, 40191 Düsseldorf (DE)
(72) Erfinder: SEIDEL, Kurt, D-4000 Düsseldorf 13 (DE); MÜLLER, Reinhard, D-5140 Erkelenz 7 (DE); HOLLENBERG, Detlef, D-4006 Erkrath (DE); KACZICH, Anke, D-4000 Düsseldorf 13 (DE)
(86) Internationale Anmeldenummer: EP9301629
(87) Internationale Veröffentlichungsnummer: WO9407456

(56) Entgegenhaltungen:
- WO-A-89/04164
- US-A- 4 375 480

## Beschreibung

Die Erfindung betrifft Zubereitungen zur Haarbehandlung mit einer speziellen Wirkstoffkombination.

Die Reinigung und Pflege der Haare ist ein wichtiger Bestandteil der menschlichen Körperpflege. Sowohl die Reinigung der Haare mit Shampoos als auch die dekorative Gestaltung der Frisur beispielsweise durch Färben oder Dauerwellen sind Eingriffe, die die natürliche Struktur und die Eigenschaften der Haare beeinflussen. So können anschließend an eine solche Behandlung beispielsweise die Naß- und Trockenkämmbarkeit des Haares, der Halt und die Fülle des Haares unbefriedigend sein oder die Haare einen erhöhten Spliß aufweisen. Weiterhin ist die gleichmäßige Verteilung der mit Haarfärbemitteln aufgebrachten Farbstoffe häufig problematisch.

Es ist daher seit langem üblich, die Haare einer speziellen Nachbehandlung zu unterziehen. Dabei werden, üblicherweise in Form einer Spülung, die Haare mit speziellen Wirkstoffen, beispielsweise quaternären Ammoniumsalzen oder speziellen Polymeren, behandelt. Durch diese Behandlung werden je nach Formulierung Kämmbarkeit, Halt und Fülle der Haare verbessert und die Splißrate verringert.

Weiterhin wurden in jüngster Zeit sogenannte Kombinationspräparate entwickelt, um den Aufwand der üblichen mehrstufigen Verfahren, insbesondere bei der direkten Anwendung durch Verbraucher, zu verringern.

Diese Präparate enthalten neben den üblichen Komponenten, beispielsweise zur Reinigung der Haare, zusätzlich Wirkstoffe, die früher den Haarnachbehandlungsmitteln vorbehalten waren. Der Konsument spart somit einen Anwendungsschritt; gleichzeitig wird der Verpackungsaufwand verringert, da ein Produkt weniger gebraucht wird.

Die zur Verfügung stehenden Wirkstoffe sowohl für separate Nachbehandlungsmittel als auch für Kombinationspräparate können noch nicht alle Wünsche der Verbraucher erfüllen. Insbesondere wird weiter nach Wirkstoffen und Wirkstoffkombinationen mit höherer Wirksamkeit bei gleichzeitig guter biologischer Abbaubarkeit gesucht.

Es wurde nun überraschenderweise gefunden, daß Mittel mit einer Kombination dreier bereits für die Haarbehandlung bekannter Wirkstoffklassen die gestellten Anforderungen in hervorragender Weise erfüllen. Insbesondere wird eine sehr gute Naß- und Trockenkämmbarkeit erreicht. Bei colorierten Haaren wird zusätzlich ein starker Egalisierungseffekt unter überraschend hoher Farbschonung erzielt. Schließlich lassen sich mit dieser Wirkstoffkombination Zubereitungen mit einer sehr guten biologischen Abbaubarkeit formulieren.

Gegenstand der Erfindung ist daher eine wäßrige Zubereitung zur Behandlung von Haaren enthaltend übliche kosmetische Bestandteile, dadurch gekennzeichnet, daß
- alkoxylierte Wollwachsalkohole (A),
- Ester des Glycerins mit gesättigten oder ungesättigten C₁₀-C₂₂-Fettsäuren (B) und
- Ester des Sorbitans mit gesättigten oder ungesättigten C₁₀-C₂₂-Fettsäuren (C) und/oder deren alkoxylierte Analoga
enthalten sind.

Wollwachsalkohole, die auch als Lanolinalkohole bezeichnet werden, sind komplexe Mischungen aus Sterinen und aliphatischen Alkoholen, die aus dem Wollwachs gewonnen werden. Hauptbestandteil der Wollwachsalkohole sind Cholesterin, Lanosterin und von diesen abgeleitete Sterine, C₁₈-C₃₀-n-Alkohole, C₁₆-C₂₆-iso-Alkohole, Alkandiole und iso-Alkandiole.

Durch Umsetzung dieser Mischungen mit Alkylenoxiden, insbesondere Ethylenoxid und Propylenoxid, werden die alkoxylierten Verbindungen erhalten, die u.a. als Emulgatoren, Lösungsvermittler, Feuchthaltemittel und Konditioniermittel bekannt sind. Diese Produkte stellen üblicherweise keine einheitlich alkoxylierten Verbindungen dar, sondern weisen in Abhängigkeit von dem gewählten Alkoxylierungsverfahren eine entsprechende Homologenverteilung auf. Der angegebene Alkoxylierungsgrad bezieht sich dabei jeweils auf die molaren Ausgangsmengen an Wollwachsalkohol und Alkylenoxid.

Ethoxylierte Wollwachsalkohole sind im Rahmen der Erfindung bevorzugt. Besonders bevorzugt sind dabei Ethoxylierungsgrade von 1 bis 50, insbesondere von 2 bis 30.

Als Glycerinester (B) können Mono-, Di- und Triester des Glycerins mit gesättigten oder ungesättigten C₁₀-C₂₂-Fettsäuren verwendet werden. Bevorzugte Fettsäuren sind Laurinsäure, Myristinsäure, Palmitinsäure, Stearinsäure, Ölsäure, Linolsäure und Linolensäure sowie Erucasäure.

Besonders bevorzugt sind Ester mit C₁₆- und C₁₈-Fettsäuren. Ebenfalls besonders bevorzugt sind die Ester mit ungesättigten Fettsäuren. Weiterhin sind die Monoester gegenüber den Di- und Tri-Estern bevorzugt.

Eine ganz besonders bevorzugte Verbindung (B) ist das Glycerinmonooleat.

Neben den oben genannten, chemisch eindeutig definierten Verbindungen können als Glycerinester auch solche Gemische eingesetzt werden, die beim Verarbeiten nativer nachwachsender Rohstoffe, z.B. Fetten und Ölen entstehen. In diesem Fall sind die Säurekomponenten der Ester jeweils Mischungen von Fettsäuren entsprechend den natürlichen Ausgangsprodukten. Ebenfalls erfindungsgemäß einsetzbar sind Mischungen aus Mono-, Di- und Tri-Estern, insbesondere von Mono- und Diestern.

Als Sorbitanester (C) können Mono-, Sesqui-, Di- und Triester des Sorbitans mit gesättigten oder ungesättigten C₁₀-C₂₂-Fettsäuren verwendet werden. Bevorzugte Fettsäuren sind hier ebenfalls Laurinsäure, Myristinsäure, Palmitinsäure, Stearinsäure, Ölsäure, Linolsäure und Linolensäure sowie Erucasäure.

Besonders bevorzugt sind Ester mit C₁₆- und C₁₈-Fettsäuren. Ebenfalls besonders bevorzugt sind die Ester mit ungesättigten Fettsäuren. Weiterhin sind die Mono- und Sesquiester gegenüber den Di- und Tri-Estern bevorzugt.

Ganz besonders bevorzugte Verbindungen (C) sind Sorbitanmonooleat und Sorbitansesquioleat.

Neben den oben genannten, chemisch eindeutig definierten Verbindungen können als Sorbitanester auch solche Gemische eingesetzt werden, die beim Verarbeiten nativer nachwachsender Rohstoffe, z.B. Fetten und Ölen entstehen. In diesem Fall sind die Säurekomponenten der Ester jeweils Mischungen von Fettsäuren entsprechend den natürlichen Ausgangprodukten.

Ebenfalls erfindungsgemäß einsetzbar sind die Verbindungen, die durch Umsetzung der genannten Sorbitanester (C) mit Alkylenoxiden, insbesondere Ethylenoxid und Propylenoxid, erhältlich sind. Diese Produkte stellen üblicherweise keine einheitlichen Verbindungen dar, sondern weisen in Abhängigkeit von dem gewählten Alkoxylierungsverfahren eine entsprechende Homologenverteilung auf. Der angegebene Alkoxylierungsgrad bezieht sich dabei jeweils auf die molaren Ausgangsmengen an Sorbitanester und Alkylenoxid. Ethoxylierte Sorbitanester sind im Rahmen der Erfindung bevorzugte alkoxylierte Sorbitanester. Besonders bevorzugt sind dabei Ethoxylierungsgrade von 1 bis 80, insbesondere von 5 bis 40.

Die erfindungsgemäßen Zubereitungen enthalten die alkoxylierten Wollwachsalkohole (A) bevorzugt in Mengen von 0,1 bis 5 Gew.-%, bezogen auf die gesamte Zubereitung. Die Glycerinester (B) und die Sorbitanester (C) sind bevorzugt in Mengen von 0,1 bis 3, insbesondere von 0,1 bis 2 Gew.-%, enthalten.

Für die Haarbehandlung, insbesondere für die Haarnachbehandlung, besonders gut geeignet sind Zubereitungen, die neben den obligatorischen Komponenten (A), (B) und (C) zusätzlich noch ein Konditionierungsmittel enthalten.

Als haarkonditionierende Verbindungen können beispielsweise kationische Tenside, kationische Polymere, quaternisierte und aminofunktionelle Silikonöle, Alkylamidoamine, Esterquats und Phospholipide verwendet werden.

Beispiele für die in den erfindungsgemäßen Haarbehandlungsmitteln verwendbaren kationischen Tenside sind insbesondere quartäre Ammoniumverbindungen. Bevorzugt sind Ammoniumhalogenide, insbesondere Chloride und Bromide wie Alkyltrimethylammoniumchloride, Dialkyldimethylammoniumchloride und Trialkylmethylammoniumchloride, z. B. Cetyltrimethylammoniumchlorid, Stearyltrimethylammoniumchlorid, Distearyldimethylammoniumchlorid, Lauryldimethylammoniumchlorid, Lauryldimethylbenzylammoniumchlorid und Tricetylmethylammoniumchlorid. Weitere erfindungsgemäß verwendbare kationische Tenside stellen die quaternisierten Proteinhydrolysate dar.

Kationische Polymere können ebenfalls als haarkonditionierende Wirkstoffe verwendet werden. Geeignete kationische Polymere enthalten üblicherweise ein quartäres Stickstoffatom, beispielsweise in Form einer Ammoniumgruppe. Bevorzugte kationische Polymere sind beispielsweise
- quaternisierte Celluloseether,
- Polysiloxane mit quaternären Gruppen,
- Dimethyldiallylammoniumchlorid-Polymere,
- Acrylamid-Dimethyldiallylammoniumchlorid-Copolymere,
- mit Diethylsulfat quaternierte Dimethylaminoethylmethacrylat-Vinylpyrrolidon-Copolymere,
- Vinylpyrrolidon-Methoimidazoliniumchlorid-Copolymere
- quaternierter Polyvinylalkohol
sowie die unter den Bezeichnungen
- Polyquaternium 2,
- Polyquaternium 17,
- Polyquaternium 18 und
- Polyquaternium 27 bekannten Polymeren mit quartären Stickstoffatomen in der Polymerhauptkette.

Erfindungsgemäß geeignete Silikonöle sind beispielsweise die im Handel erhältlichen Produkte Q2-7224 (Hersteller: Dow Corning; ein stabilisiertes Trimethylsilylamodimethicon), Dow Corning 929 Emulsion (enthaltend ein hydroxyl-amino-modifiziertes Silicon, das auch als Amodimethicone bezeichnet wird), SM-2059 (Hersteller: General Electric), SLM-55067 (Hersteller: Wacker) sowie Abil^{R}-Quat 3270 und 3272 (Hersteller: Th. Goldschmidt; diquaternäre Polydimethylsiloxane, Quaternium-80).

Alkylamidoamine, insbesondere Fettsäureamidoamine wie das unter der Bezeichnung Tego Amid^{R}S 18 erhältliche Stearylamidopropyldimethylamin, zeichnen sich neben einer guten konditionierenden Wirkung speziell durch ihre gute biologische Abbaubarkeit aus.

Ebenfalls sehr gut biologisch abbaubar sind die sogenannten Esterquats wie die unter dem Warenzeichen Stepantex^{R} vertriebenen Dialkylammoniummethosulfate und Methyl-hydroxyalkyl-dialkoyloxyalkyl-ammoniummethosulfate.

Schließlich können als haarkonditionierende Verbindungen auch natürliche Stoffe wie Phospholipide, beispielsweise Sojalecithin, Ei-Lecitin und Kephaline eingesetzt werden.

In Hinblick auf die biologische Abbaubarkeit der erfindungsgemäßen Zubereitungen werden als haarkonditionierende Mittel bevorzugt Alkylamidoamine, Esterquats und Phospholipide eingesetzt. Die Verwendung von Alkylamidoaminen ist besonders bevorzugt.

Die erfindungsgemäßen Zubereitungen weisen bevorzugt einen pH-Wert zwischen 3,5 und 6, insbesondere zwischen 4,0 und 5,0 auf. Lediglich bei speziellen Produkten wie Färbemitteln und Dauerwellmitteln können höhere pH-Werte, in der Regel aber nicht oberhalb von 10, bevorzugt sein.

Zur Einstellung dieses pH-Wertes kann praktisch jede für kosmetische Zwecke verwendbare Säure verwendet werden. Üblicherweise, insbesonders wenn es sich bei der Zubereitung nicht um ein Wellmittel handelt, werden Genußsäuren verwendet. Unter Genußsäuren werden solche Säuren verstanden, die im Rahmen der üblichen Nahrungsaufnahme aufgenommen werden und positive Auswirkungen auf den menschlichen Organismus haben. Genußsäuren sind beispielsweise Essigsäure, Milchsäure, Weinsäure, Zitronensäure, Äpfelsäure, Ascorbinsäure und Gluconsäure. Im Rahmen der Erfindung ist die Verwendung von Zitronensäure und Milchsäure besonders bevorzugt.

Weiterhin können die erfindungsgemäßen Zubereitungen alle für den jeweiligen Verwendungszweck üblichen kosmetischen Zusätze enthalten.

So können die Zubereitungen beispielsweise als Haarnachbehandlungsmittel, Shampoo, Haarfärbemittel und Wellmittel formuliert sein.

Die Formulierung der Zubereitungen als Haarnachbehandlungsmittel, insbesondere als Spülung, ist im Rahmen der erfindungsgemäßen Lehre bevorzugt. In diesem Fall wird die Zubereitung neben den bereits genannten Komponenten im wesentlichen noch Wasser, Parfümöle, Lösungsvermittler und Farbstoffe enthalten.

Eine Formulierung als Shampoo kommt vor allem in Hinblick auf die sogenannten Kombinationspräparate in Betracht, die Haarreinigungsmittel und Haarnachbehandlungsmittel in einer Zubereitung vereinigen. Neben der Basis Wasser enthalten diese Zubereitungen als wichtige Komponente oberflächenaktive Verbindungen, die ausgewählt sind aus der Gruppe der anionischen, zwitterionischen, amphoteren und nichtionischen Tenside.

Als anionische Tenside eignen sich in erfindungsgemäßen Zubereitungen alle für die Verwendung am menschlichen Körper geeigneten anionischen oberflächenaktiven Stoffe. Diese sind gekennzeichnet durch eine wasserlöslich machende, anionische Gruppe wie z. B. eine Carboxylat-, Sulfat-, Sulfonat- oder Phosphat-Gruppe und eine lipophile Alkylgruppe mit etwa 10 bis 22 C-Atomen. Zusätzlich können im Molekül Glykol- oder Polyglykolether-Gruppen, Ester-, Ether- und Amidgruppen sowie Hydroxylgruppen enthalten sein. Beispiele für geeignete anionische Tenside sind, jeweils in Form der Natrium-, Kalium- und Ammonium- sowie der Mono-, Di- und Trialkanolammoniumsalze mit 2 oder 3 C-Atomen in der Alkanolgruppe,
- lineare Fettsäuren mit 10 bis 22 C-Atomen (Seifen),
- Ethercarbonsäuren der Formel R-O-(CH₂-CH₂O)ₓ-CH₂-COOH, in der R eine lineare Alkylgruppe mit 10 bis 22 C-Atomen und x = 0 oder 1 bis 16 ist,
- Acylsarcoside mit 10 bis 18 C-Atomen in der Acylgruppe,
- Acyltauride mit 10 bis 18 C-Atomen in der Acylgruppe,
- Acylisethionate mit 10 bis 18 C-Atomen in der Acylgruppe,
- Sulfobernsteinsäuremono- und dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen,
- lineare Alkansulfonate mit 12 bis 18 C-Atomen,
- lineare Alpha-Olefinsulfonate mit 12 bis 18 C-Atomen,
- Alpha-Sulfofettsäuremethylester von Fettsäuren mit 12 bis 18 C-Atomen,
- Alkylsulfate und Alkylpolyglykolethersulfate der Formel R-O(CH₂-CH₂O)ₓ-OSO₃H, in der R eine bevorzugt lineare Alkylgruppe mit 10 bis 18 C-Atomen und x = 0 oder 1 bis 12 ist,
- Gemische oberflächenaktiver Hydroxysulfonate gemäß DE-A-37 25 030,
- sulfatierte Hydroxyalkylpolyethylen- und/oder Hydroxyalkylenpropylenglykolether gemäß DE-A-37 23 354,
- Sulfonate ungesättigter Fettsäuren mit 12 bis 24 C-Atomen und 1 bis 6 Doppelbindungen gemäß DE-A-39 26 344,
- Ester der Weinsäure und Zitronensäure mit Alkoholen, die Anlagerungsprodukte von etwa 2-15 Molekülen Ethylenoxid und/oder Propylenoxid an Fettalkohole mit 8 bis 22 C-Atomen darstellen.

Bevorzugte anionische Tenside sind Alkylsulfate, Alkylpolyglykolethersulfate und Ethercarbonsäuren mit 10 bis 18 C-Atomen in der Alkylgruppe und bis zu 12 Glykolethergruppen im Molekül, sowie Sulfobernsteinsäuremono- und -dialkylester mit 8 bis 18 C-Atomen in der Alkylgruppe und Sulfobernsteinsäuremono-alkylpolyoxyethylester mit 8 bis 18 C-Atomen in der Alkylgruppe und 1 bis 6 Oxyethylgruppen.

Als zwitterionische Tenside werden solche oberflächenaktiven Verbindungen bezeichnet, die im Molekül mindestens eine quartäre Ammoniumgruppe und mindestens eine -COO⁽⁻⁾- oder -SO₃⁽⁻⁾-Gruppe tragen. Besonders geeignete zwitterionische Tenside sind die sogenannten Betaine wie die N-Alkyl-N,N-dimethylammonium-glycinate, beispielsweise das Kokosalkyl-dimethylammoniumglycinat, N-Acyl-aminopropyl-N,N-dimethylammoniumglycinate, beispielsweise das Kokosacylaminopropyl-dimethylammoniumglycinat, und 2-Alkyl-3-carboxymethyl-3-hydroxyethyl-imidazoline mit jeweils 8 bis 18 C-Atomen in der Alkyl- oder Acylgruppe sowie das Kokosacylaminoethylhydroxyethylcarboxymethylglycinat. Ein bevorzugtes zwitterionisches Tensid ist das unter der CTFA-Bezeichnung Cocamidopropyl Betaine bekannte Fettsäureamid-Derivat.

Unter ampholytischen Tensiden werden solche oberflächenaktiven Verbindungen verstanden, die außer einer C₈-C₁₈-Alkyl- oder -Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe. Besonders bevorzugte ampholytische Tenside sind das N-Kokosalkylaminopropionat, das Kokosacylaminoethylaminopropionat und das C₁₂₋₁₈-Acylsarcosin.

Nichtionogene Tenside enthalten als hydrophile Gruppe z. B. eine Polyolgruppe, eine Polyalkylenglykolethergruppe oder eine Kombination aus Polyol- und Polyglykolethergruppe. Solche Verbindungen sind beispielsweise
- Anlagerungsprodukte von 2 bis 30 Mol Ethylenoxid und/oder 0 bis 5 Mol Propylenoxid an lineare Fettalkohole mit 8 bis 22 C-Atomen, an Fettsäuren mit 12 bis 22 C-Atomen und an Alkylphenole mit 8 bis 15 C-Atomen in der Alkylgruppe,
- C₁₂-C₂₂-Fettsäuremono- und -diester von Anlagerungsprodukten von 1 bis 30 Mol Ethylenoxid an Glycerin,
- C₈-C₂₂-Alkylmono- und oligoglycoside und deren ethoxylierte Analoga sowie
- Anlagerungsprodukte von 5 bis 60 Mol Ethylenoxid an Rizinusöl und gehärtetes Rizinusöl.

Bei den als Tenside eingesetzten Verbindungen mit Alkylgruppen kann es sich jeweils um einheitliche Substanzen handeln. Es ist jedoch in der Regel bevorzugt, bei der Herstellung dieser Stoffe von nativen pflanzlichen oder tierischen Rohstoffen auszugehen, so daß man Substanzgemische mit unterschiedlichen, vom jeweiligen Rohstoff abhängigen Alkylkettenlängen erhält.

Bei den Tensiden, die Anlagerungsprodukte von Ethylen- und/oder Propylenoxid an Fettalkohole oder Derivate dieser Anlagerungsprodukte darstellen, können sowohl Produkte mit einer "normalen" Homologenverteilung als auch solche mit einer eingeengten Homologenverteilung verwendet werden. Unter "normaler" Homologenverteilung werden dabei Mischungen von Homologen verstanden, die man bei der Umsetzung von Fettalkohol und Alkylenoxid unter Verwendung von Alkalimetallen, Alkalimetallhydroxiden oder Alkalimetallalkoholaten als Katalysatoren erhält. Eingeengte Homologenverteilungen werden dagegen erhalten, wenn beispielsweise Hydrotalcite, Erdalkalimetallsalze von Ethercarbonsäuren, Erdalkalimetalloxide, -hydroxide oder -alkoholate als Katalysatoren verwendet werden. Die Verwendung von Produkten mit eingeengter Homologenverteilung kann bevorzugt sein.

Vorzugsweise enthalten die erfindungsgemäßen Zubereitungen die oberflächenaktiven Verbindungen A in Mengen von 0,5 bis 20 Gew.-%, insbesondere von 0,5 bis 10 Gew.-%, bezogen auf die jeweilige Zubereitung.

Wegen der egalisierenden Eigenschaften der erfindungsgemäßen Wirkstoffkombination ist auch eine Formulierung der Zubereitung als Haarfärbemittel vorteilhaft. In diesem Fall enthalten die Zubereitungen als zwingende Komponenten direktziehende Farbstoffe und/oder Vorprodukte zur Bildung von Oxidationsfarbstoffen. Die entsprechenden, für den Gebrauch am menschlichen Haar geeigneten Farbstoffe und Farbstoffvorprodukte sind dem Fachmann bekannt.

Bei Formulierung der erfindungsgemäßen Zubereitungen als Dauerwellmittel können die Zubereitungen sowohl als Wellösung zur Durchführung der reduzierenden ersten Stufe des Dauerwellverfahrens als auch als Fixierlösung formuliert sein. Wellösungen enthalten als zwingende Komponenten keratinreduzierende Verbindungen wie Thioglykolsäure und deren physiologisch unbedenkliche Salze, Thiomilchsäure, Cysteamin, Thioäpfelsäure und α-Mercaptoethansulfonsäure. Fixierlösungen sind üblicherweise sauer eingestellt und enthalten Oxidationsmittel wie beispielsweise Kaliumbromat oder Wasserstoffperoxid. Im Rahmen einer Dauerwellbehandlung können sowohl Wellösung als auch Fixierlösung die erfindungsgemäße Wirkstoffkombination enthalten. Gleichwohl wird bereits ein Großteil des Effektes erzielt, wenn lediglich eine der beiden Lösungen, insbesondere die Fixierlösung, diese Wirkstoffkombination enthält.

Weitere übliche Bestandteile der erfindungsgemäßen Zubereitungen können sein:
- Anionische, zwitterionische, amphotere und nichtionische Polymere wie beispielsweise Vinylacetat/Crotonsäure-Copolymere,
   Vinylpyrrolidon/Vinylacrylat-Copolymere, Vinylacetat/Butylmaleat/Isobornylacrylat-Copolymere,
   Methylvinylether/Maleinsäureanhydrid-Copolymere und deren Ester, unvernetzte und mit Polyolen vernetzte Polyacrylsäuren, Acrylamidopropyl-trimethylammoniumchlorid/Acrylat-Copolymere, Octylacrylamid/Methyl-methacrylat/tert.Butylaminoethylmethacrylat/2-Hydroxypropylmethacrylat-Copolymere, Polyvinylpyrrolidon, Vinylpyrrolidon/Vinylacetat-Copolymere, Vinylpyrrolidon/Dimethylaminoethylmethacrylat/Vinylcaprolactam-Terpolymere sowie gegebenfalls derivatisierte Celluloseether.
- Verdickungsmittel wie Agar-Agar, Guar-Gum, Alginate und Xanthan-Gum,
- Strukturanten wie Glucose und Maleinsäure,
- Proteinhydrolysate, insbesondere Elastin-, Kollagen-, Keratin-, Milcheiweiß-, Sojaprotein- und Weizenproteinhydrolysate, deren Kondensationsprodukte mit Fettsäuren sowie quaternisierte Proteinhydrolysate,
- Parfümöle, Dimethylisosorbid und Cyclodextrine,
- Lösungsvermittler, wie Ethanol, Isopropanol, Ethylenglykol, Propylenglykol, Glycerin und Diethylenglykol,
- Farbstoffe,
- Antischuppenwirkstoffe wie Piroctone Olamine und Zink Omadine,
- weitere Substanzen zur Einstellung des pH-Wertes
- Wirkstoffe wie Panthenol, Allantoin, Pyrrolidoncarbonsäuren, Pflanzenextrakte und Vitamine,
- Lichtschutzmittel,
- Konsistenzgeber wie Zuckerester, Polyolester oder Polyolalkylether,
- Fette und Wachse, wie Walrat, Bienenwachs, Montanwachs, Paraffine und Fettalkohole,
- Fettsäurealkanolamide,
- Komplexbildner wie EDTA, NTA und Phosphonsäuren,
- Quell- und Penetrationsstoffe wie Glycerin, Propylenglykolmonoethylether, Carbonate, Hydrogencarbonate, Guanidine, Harnstoffe sowie primäre, sekundäre und tertiäre Phosphate,
- Trübungsmittel wie Latex,
- Perlglanzmittel wie Ethylenglykolmono- und -distearat,
- Treibmittel wie Propan-Butan-Gemische, N₂O, Dimethylether, CO₂ und Luft sowie
- Antioxidantien.

Gegenstand der Erfindung ist auch ein Verfahren zur Behandlung von Haaren mit einer Zubereitung gemäß einem der Ansprüche 1 bis 12. Besonders bevorzugt sind solche Verfahren, bei denen die Haare nach der Behandlung mit Wasser oder einem im wesentlichen aus Wasser bestehenden Mittel gespült werden.

Die folgenden Beispiele sollen den Erfindungsgegenstand näher erläutern.

### Beispiele

### I. Farbmetrische Untersuchungen zur Farberhaltung bei colorierten Haaren

### Untersuchungsmethode

Eine Egalisiersträhne (naturweiß, 2 g, ca. 15 cm lang; Fa. Kerling) wurde auf halber Höhe abgebunden. Zur Simulation von stark geschädigtem Haar wurde der untere Teil der Strähne abwechselnd je zweimal kaltgewellt und ultrablondiert. Die Kaltwellung erfolgte jeweils durch Behandlung mit einer wäßrigen Lösung eines Kaltwellmittels auf Basis Ammoniumthioglykolat (30 Minuten) und anschließende Fixierung mit Kaliumbromat-Lösung (10 Minuten). Die Ultrablondierung erfolgt mit einer wäßrigen Lösung von Wasserstoffperoxid und Ammoniumperoxidisulfat. Der obere Teil wurde zur Simulation von wenig geschädigtem Haar lediglich einmal ultrablondiert. Anschließend wurde die gesamte Strähne mit dem Handelsprodukt Poly Diadem Pflege Intensiv Tönung (Nuance Mahagoni-Koralle) (HENKEL) ausgefärbt. Dabei wurden 4 g Färbemischung pro 1 g Haarsträhne eingesetzt. Nach einer Einwirkzeit von 30 Minuten wurden die Strähne mit warmem Wasser (30 °C) gut ausgespült und mit einem Fön getrocknet. Die ausgefärbte Haarsträhne wurde dann 4 Tage bei Raumtemperatur gelagert.

Nach der farbmetrischen Bestimmung des Nullwertes wurde die Haarsträhne 2 Minuten mit 0,5 g Testmischung pro 2 g Haar behandelt. Anschließend wurde die Strähne mit Wasser (30 °C) gründlich gespült, getrocknet und farbmetrisch vermessen. Die Messung wurde mit einem Gerät Texflash (Fa. Datacolor) mit Lichtart D65 (Tageslicht) durchgeführt. Dabei wurde die zu vermessende Probe in einer Einspannvorrichtung am Spektralphotometer fixiert und die Remissionswerte über den Bereich des sichtbaren Lichtes von 390 - 700 nm im Abstand von 10 nm gemessen und über einen Rechner verarbeitet. Das Rechnerprogramm ermittelte die Normfarbwerte nach dem CIE-System (Commission Internationale de L'Eclairage) entsprechend DIN 5033 und rechnete sie in Farbabstandszahlen nach DIN 6174 um. Die im weiteren angegebenen Meßwerte sind jeweils Mittelwerte aus 4 Meßpunkten pro Strähnenteil.

Es wurden folgende Größen bestimmt:
- Gesamtfarbabstand DE: (relativ zum Nullwert)
- Egalisierung E: (Differenz der Gesamtfarbabstände DE nach der Behandlung von stark und wenig geschädigtem Haar)

Die Zusammensetzungen der untersuchten Mischungen und die Meßergebnisse sind in den Tabellen 1 und 2 aufgeführt.

**Tabelle 1**

| **[Mengenangaben in Gewichtsteilen]** | | | | | | | | | | | |
|---|---|---|---|---|---|---|---|---|---|---|---|
| Komponente / Mischung | A1 | A2 | A3 | A4 | A5 | A6 | A7 | A8 | A9 | A10 | A11 |
| Stenol^{R} 1618¹ | 2,5 | 2,5 | 2,5 | 2,5 | 2,5 | 2,5 | 2,5 | 2,5 | 2,5 | 2,5 | 2,5 |
| Paraffinöl perliq | 2,5 | 2,5 | 2,5 | 2,5 | 2,5 | 2,5 | 2,5 | 2,5 | 2,5 | 2,5 | 2,5 |
| Dehydol^{R} 100² | 0,2 | 0,2 | 0,2 | 0,2 | 0,2 | 0,2 | 0,2 | 0,2 | 0,2 | 0,2 | 0,2 |
| Dehyquart^{R} A³ | 2,4 | 2,4 | 2,4 | 2,4 | 2,4 | 2,4 | 2,4 | 2,4 | 2,4 | 2,4 | 2,4 |
| Polychol^{R} 5⁴ | 0,5 | - | 0,5 | 1,0 | - | - | - | - | 0,5 | 0,5 | - |
| Dehymuls^{R} SSO⁵ | 0,5 | - | - | - | 0,5 | 1,0 | - | - | 0,5 | - | 0,5 |
| Monomuls^{R} 90 018⁶ | 0,5 | - | - | - | - | - | 0,5 | 1,0 | - | 0,5 | 0,5 |
| Parfümöl, Konservierungsmittel, Wasser | <-----------------------------ad 100 -----------------------> | | | | | | | | | | |
| DE (wenig geschädigtes Haar) | 2,6 | 4,5 | 4,0 | 3,7 | 4,1 | 3,9 | 3,9 | 3,8 | 3,2 | 3,1 | 3,4 |
| DE (stark geschädigtes Haar) | 5,9 | 9,1 | 8,1 | 7,7 | 8,3 | 7,8 | 7,9 | 7,5 | 7,1 | 6,7 | 7,0 |
| E | 3,7 | 6,9 | 4,1 | 4,5 | 4,2 | 4,5 | 4,0 | 4,2 | 5,0 | 4,7 | 4,0 |

| | | | | | | | | | | | |
|---|---|---|---|---|---|---|---|---|---|---|---|
| ¹ C₁₆/C₁₈-Fettalkohol (HENKEL) | | | | | | | | | | | |
| ² C₁₂/C₁₈-Fettalkohol mit 9 Mol Ethylenoxid (CTFA-Bezeichnung: Laureth-10) (HENKEL) | | | | | | | | | | | |
| ³ Trimethylhexadecylammoniumchlorid (25 % Aktivsubstanz; CTFA-Bezeichnung: Cetrimoniumchlorid) (Henkel) | | | | | | | | | | | |
| ⁴ Lanolinalkohol + 5 Ethylenoxid (CTFA-Bezeichnung: Laneth-5) (CRODA) | | | | | | | | | | | |
| ⁵ Sorbitansesquioleat (HENKEL) | | | | | | | | | | | |
| ⁶ Glycerinmonooleat (CTFA-Bezeichnung: Glyceryl Oleate) (HENKEL) | | | | | | | | | | | |

**Tabelle 2**

| **[Mengenangabe in Gewichtsteilen]** | | | | | | | | | | | |
|---|---|---|---|---|---|---|---|---|---|---|---|
| Komponente / Mischung | B1 | B2 | B3 | B4 | B5 | B6 | B7 | B8 | B9 | B10 | B11 |
| Stenol^{R} 1618 | 1,8 | 1,8 | 1,8 | 1,8 | 1,8 | 1,8 | 1,8 | 1,8 | 1,8 | 1,8 | 1,8 |
| Tegoamid^{R} S 18⁷ | 1,6 | 1,6 | 1,6 | 1,6 | 1,6 | 1,6 | 1,6 | 1,6 | 1,6 | 1,6 | 1,6 |
| Cutina^{R} CP⁸ | 1,0 | 1,0 | 1,0 | 1,0 | 1,0 | 1,0 | 1,0 | 1,0 | 1,0 | 1,0 | 1,0 |
| 1,2-Propylenglykol | 2,0 | 2,0 | 2,0 | 2,0 | 2,0 | 2,0 | 2,0 | 2,0 | 2,0 | 2,0 | 2,0 |
| Milchsäure | 0,5 | 0,5 | 0,5 | 0,5 | 0,5 | 0,5 | 0,5 | 0,5 | 0,5 | 0,5 | 0,5 |
| Polychol^{R} 5 | 0,5 | - | 0,5 | 1,0 | - | - | - | - | 0,5 | 0,5 | - |
| Dehymuls^{R} SMO⁹ | 0,5 | - | - | - | 0,5 | 1,0 | - | - | 0,5 | - | 0,5 |
| Monomuls^{R} 90 0 18 | 0,5 | - | - | - | - | - | 0,5 | 1,0 | - | 0,5 | 0,5 |
| Parfümöl, Konservierungsmittel, Wasser | <----------------------------------ad 100 -----------------------------> | | | | | | | | | | |
| DE (wenig geschädigtes Haar) | 2,5 | 4,7 | 4,2 | 3,8 | 4,1 | 3,5 | 4,1 | 3,9 | 3,1 | 3,5 | 3,3 |
| DE (stark geschädigtes Haar) | 3,7 | 9,5 | 7,5 | 6,8 | 7,8 | 7,0 | 7,6 | 6,7 | 5,4 | 5,7 | 5,5 |
| E | 3,2 | 7,6 | 3,3 | 5,4 | 3,7 | 6,3 | 3,5 | 4,3 | 4,4 | 4,3 | 4,0 |
| alle Mischungen wurden mit Zitronensäure auf pH = 4,0 eingestellt | | | | | | | | | | | |

| | | | | | | | | | | | |
|---|---|---|---|---|---|---|---|---|---|---|---|
| ⁷ N,N-Dimethyl-N'-stearoyl-1,3-diamino-propan (CTFA-Bezeichnung: Stearamidopropyl Dimethylamin (GOLDSCHMIDT) | | | | | | | | | | | |
| ⁸ Ester aus gesättigten, langkettigen Fettalkoholen und Fettsäuren, vornehmlich Palmitinsäurecetylester (CTFA-Bezeichnung: Cetyl Palmitate) (HENKEL) | | | | | | | | | | | |
| ⁹ Sorbitanmonooleat (HENKEL) | | | | | | | | | | | |

### II. Anwendungsbeispiele

Die Mengenangaben in den folgenden Beispielen sind Gew.-%.

### 1) Haarspülung

| | |
|---|---|
| Stenol^{R}1618 | 1,8 |
| Tegoamid^{R}S 18 | 1,6 |
| 1,2-Propylenglykol | 1,0 |
| Zitronensäure | 0,6 |
| Polychol^{R}5 | 0,5 |
| Dehymuls^{R}SSO | 0,15 |
| Monomuls^{R}90 0 18 | 0,2 |
| Parfümöl, Wasser | ad 100 |
| Der pH-Wert der Spülung betrug 3,8. | |

### 2) Haarspülung

| | |
|---|---|
| Stenol^{R}1618 | 2,0 |
| Tegoamid^{R}S 18 | 1,5 |
| 1,2-Propylenglykol | 1,2 |
| Milchsäure | 0,5 |
| Polychol^{R}5 | 0,8 |
| Dehymuls^{R}SMO | 0,3 |
| Monomuls^{R}90 0 18 | 0,5 |
| Parfümöl, Wasser | ad 100 |
| Der pH-Wert der Spülung betrug 4,0. | |

### 3) Haarspülung

| | |
|---|---|
| Stenol^{R}1618 | 1,8 |
| Stepantex^{R} VS 90¹⁰ | 1,8 |
| 1,2-Propylenglykol | 0,7 |
| Zitronensäure | 0,9 |
| Polychol^{R}5 | 0,5 |
| Dehymuls^{R}SSO | 0,3 |
| Monomuls^{R}90 0 18 | 0,3 |
| Parfümöl, Farbstoff, Wasser | ad 100 |
| Der pH-Wert der Spülung betrug 4,8. | |

| | |
|---|---|
| ¹⁰ N-Methyl-N(2-hydroxyethyl)-N,N-di(talgacyloxyethyl)ammoniummethosulfat (90% Aktivsubstanz in Isopropanol) (STEPAN) | |

### 4) Haarspülung

| | |
|---|---|
| Stenol^{R}1618 | 2,5 |
| Eumulgin^{R}B 1¹¹ | 1,0 |
| Eumulgin^{R}B 2¹² | 1,0 |
| Cutina^{R}CP | 1,0 |
| Eutanol^{R}G¹³ | 0,5 |
| Polawax^{R}GP 200¹⁴ | 0,75 |
| Abil^{R}Quat 3270¹⁵ | 0,5 |
| Dow Corning^{R}929-Emulsion¹⁶ | 2,6 |
| Polychol^{R}5 | 1,0 |
| Dehymuls^{R}SMO | 0,6 |
| Monomuls^{R}90 0 18 | 0,5 |
| Zitronensäure | 0,02 |
| Wasser | ad 100 |
| Der pH-Wert der Spülung betrug 4,0. | |

| | |
|---|---|
| ¹¹ Cetylstearylalkohol mit ca. 12 Mol EO (CTFA-Bezeichnung: Ceteareth12) (HENKEL) | |
| ¹² Cetylstearylalkohol mit ca. 20 Mol EO (CTFA-Bezeichnung: Ceteareth-20) (HENKEL) | |
| ¹³ Kondensationsprodukt aus gesättigten flüssigen Fettalkoholen, vorwiegen Decylalkohol, hergestellt nach der Guerbet-Reaktion (CTFA-Bezeichnung: Octyldodecanol) (HENKEL) | |
| ¹⁴ Stearylalkohol-Polyethylenglykolstearat-Gemisch (CTFA-Bezeichnung: Stearyl Alkohol (and) PEG-Stearate) (CRODA) | |
| ¹⁵ Diquaternäres Polymethylsiloxane (CTFA-Bezeichnung: Quaternium-80) (GOLDSCHMIDT) | |
| ¹⁶ amino-funktionelles Polydimethylsiloxan (35 % Aktivsubstanz) (DOW CORNING) | |

### 5) Haarspülung

| | |
|---|---|
| Rewoquat^{R}W 7500¹⁷ | 1,1 |
| Lanette O¹⁸ | 3,0 |
| Eumulgin^{R}B 1 | 0,8 |
| Eumulgin^{R}B 2 | 1,6 |
| Cutina^{R}GMS¹⁹ | 0,5 |
| Eutanol^{R}G | 1,0 |
| Polychol^{R}5 | 2,0 |
| Dehymuls^{R}SMS²⁰ | 0,7 |
| Monomuls^{R}90 0 18 | 1,5 |
| Wasser | ad 100 |

| | |
|---|---|
| ¹⁷ 1-Methyl-2-nortalgalkyl-3-talgfettsäure-amidoethyl-imidazoliniummethosulfat (ca. 75 % Aktivsubstanz) (REWO) | |
| ¹⁸ Gemisch höherer, gesättigter Fettalkohole, vorwiegend Cetyl- und Stearylalkohol (CTFA-Bezeichnung: Cetearyl Alcohol) (HENKEL) | |
| ¹⁹ Glycerinmonostearat (CTFA-Bezeichnung: Glyceryl Stearate) (HENKEL) | |
| ²⁰ Sorbitanmonostearat (CTFA-Bezeichnung: Sorbitan Stearate) (HENKEL) | |

### 6) Haarspülung

| | |
|---|---|
| Stenol^{R}1618 | 2,5 |
| Paraffinöl perliq. | 2,5 |
| Dehydol^{R}100 | 0,5 |
| Dehyquart^{R}A | 2,4 |
| Polychol^{R}5 | 3,0 |
| Dehymuls^{R}SSO | 0,8 |
| Monomuls^{R}90 0 18 | 1,0 |
| Wasser | ad 100 |
| Der pH-Wert der Spülung betrug 4,5. | |

### 7) Haarspülung

| | |
|---|---|
| Stenol^{R}1618 | 3,5 |
| Cutina^{R}GMS | 1,0 |
| Eutanol^{R}G | 2,0 |
| Eumulgin^{R}B 3²¹ | 1,4 |
| Span^{R}20²² | 1,6 |
| Akypoquat^{R}131²³ | 0,7 |
| Dow Corning^{R}200 Fluid, 0,65mm²s⁻¹ ²⁴ | 1,0 |
| Polychol^{R}5 | 1,5 |
| Dehymuls^{R}SMS¹⁸ | 0,7 |
| Monomuls^{R}90 0 18 | 1,0 |
| Milchsäure | 0,8 |
| Wasser | ad 100 |
| Der pH-Wert der Spülung betrug 3,5. | |

| | |
|---|---|
| ²¹ Cetylstearylalkohol mit ca. 30 Mol Ethylenoxid (CTFA-Bezeichnung: Ceteareth-30) (HENKEL) | |
| ²² Sorbitanmonolaurat (ATLAS) | |
| ²³ Quartäre Ammoniumverbindung auf Basis Trimethylamin-Epichlorhydrin-Behensäure (70 % Aktivsubstanz) (CHEM-Y) | |
| ²⁴ Hexamethyl-Disiloxan (CTFA-Bezeichnung: Hexamethyl-Bisiloxane) (DOW CORNING) | |

### 8) Shampoo

| | |
|---|---|
| Texapon^{R}N 25²⁵ | 43,0 |
| Dehyton^{R}K²⁶ | 10,0 |
| Plantaren^{R}-1200²⁷ | 4,0 |
| Euperlan^{R}PK 3000²⁸ | 1,6 |
| Arquad^{R}316²⁹ | 0,8 |
| Polychol^{R}20³⁰ | 3,0 |
| Dehymuls^{R}SML³¹ | 1,5 |
| Monomuls^{R}90 0 18 | 1,0 |
| Glucamate^{R}DOE 120³² | 0,5 |
| Natriumchlorid | 0,2 |
| Wasser | ad 100 |

| | |
|---|---|
| ²⁵ Natriumlaurylethersulfat (ca. 28 % Aktivsubstanz; CTFA-Bezeichnung: Sodium Laureth Sulfate) (HENKEL) | |
| ²⁶ Fettsäureamid-Derivat mit Betainstruktur der Formel R-CONH(CH₂)₃N⁺(CH₃)₂CH₂COO⁻ (ca. 30 % Aktivsubstanz; CTFA-Bezeichnung Cocoamidopropyl Betaine) (HENKEL) | |
| ²⁷ C₁₂-C₁₆-Alkylglucosid mit Oligomerisationsgrad 1,4 (ca. 50 % Aktivsubstanz; CTFA-Bezeichnung: Lauryl Polyglycosid) (HENKEL) | |
| ²⁸ Flüssige Dispersion von perlglanzgebenden Substanzen und Amphotensid (ca. 62 % Aktivsubstanz; CTFA-Bezeichnung: Glycol Distearate (and) Glycerin (and) Laureth-4 (and) Cocoamidopropyl Betaine) (HENKEL) | |
| ²⁹ Tri-C₁₆-alkylmethylammoniumchlorid (AKZO) | |
| ³⁰ Lanolinalkohol + 20 Ethylenoxid (CTFA-Bezeichnung: Laneth-20) (CRODA) | |
| ³¹ Sorbitanmonolaurat (HENKEL) | |
| ³² ethoxyliertes Methylglucosid-dioleat (CTFA-Bezeichnung: PEG-120 Methyl Glucose Dioleate) (AMERCHOL) | |

### 9) Shampoo

| | |
|---|---|
| Texapon^{R}N 70³³ | 21,0 |
| Plantaren^{R}-1200 | 8,0 |
| Genamin^{R}DSAC³⁴ | 1,2 |
| Cutina^{R}EGMS³⁵ | 0,6 |
| Polychol^{R}20 | 2,0 |
| Dehymuls^{R}SMO | 2,0 |
| Monomuls^{R}90 0 18 | 1,5 |
| Antil ^{R}141³⁶ | 1,3 |
| Natriumchlorid | 0,2 |
| Wasser | ad 100 |

| | |
|---|---|
| ³³ Natriumlaurylethersulfat (ca. 72 % Aktivsubstanz) (HENKEL) | |
| ³⁴ Dimethyldistearylammoniumchlorid (HOECHST) | |
| ³⁵ Ethylenglykolmonostearat (ca. 25-35% Monoester, 60-70% Diester; CTFA-Bezeichnung: Glycol Stearate) (HENKEL) | |
| ³⁶ Polyoxyethylen-propylenglykoldioleat (40 % Aktivsubstanz; CTFA-Bezeichnung: Propylene Glycol (and) PEG-55 Propylene Glycol Oleate) (GOLDSCHMIDT) | |

### 10) Shampoo

| | |
|---|---|
| Texapon^{R}K 14 S³⁷ | 50,0 |
| Dehyton^{R}K | 10,0 |
| Akypo^{R}RLM 100 NV³⁸ | 4,5 |
| Polymer P1, entsprechend DE 39 29 973* | 0,6 |
| Cutina^{R}AGS³⁹ | 2,0 |
| D-Panthenol | 0,5 |
| Glucose | 1,0 |
| Salicylsäure | 0,4 |
| Natriumchlorid | 0,5 |
| Polychol^{R}20 | 2,5 |
| Dehymuls^{R}SSO | 1,0 |
| Monomuls^{R}90 0 18 | 2,5 |
| Wasser | ad 100 |
| Der pH-Wert des Shampoos betrug 5,2. | |

| | |
|---|---|
| * Acrylamidopropyltrimethylammoniumchlorid/Acrylsäure-Copolymeres, mit Natronlauge neutralisiert | |
| ³⁷ Natriumlaurylmyristylethersulfat (ca. 28 % Aktivsubstanz; CTFA-Bezeichnung: Sodium Myreth Sulfate) (HENKEL) | |
| ³⁸ C₁₂₋₁₄-Fettalkohol+10 Ethylenoxid-essigsäure-Natriumsalz (22 % Aktivsubstanz; CTFA-Bezeichnung: Sodium Laureth-11 Carboxylate) (CHEM-Y) | |
| ³⁹ Ethylenglykolstearat (ca. 5-15% Monoester, 85-95% Diester; CTFA-Bezeichnung: Glycol Distearate) (HENKEL) | |

### 11) Shampoo

| | |
|---|---|
| Texapon^{R}K 14 S | 25,0 |
| Texapon^{R}SB 3⁴⁰ | 7,5 |
| Eucarol^{R}TA⁴¹ | 12,0 |
| Akypo^{R}RLM 100 NV | 9,0 |
| Dehyton^{R}AB 30⁴² | 8,3 |
| Polymer P1, entsprechend DE 39 29 973 | 0,6 |
| Elfacos^{R}GT 282S⁴³ | 0,5 |
| Polychol^{R}20 | 4,0 |
| Dehymuls^{R}SMS | 1,0 |
| Monomuls^{R}90 0 18 | 1,5 |
| Natriumchlorid | 0,5 |
| Wasser | ad 100 |
| Der pH-Wert des Shampoos betrug 5,0. | |

| | |
|---|---|
| ⁴⁰ Sulfobernsteinsäurehalbester auf Basis eines Alkylpolyglycolethers, Di-Na-Salz (ca. 40 % Aktivsubstanz; CTFA-Bezeichnung: Disodium Laurethsulfosuccinat) (HENKEL) | |
| ⁴¹ Laurylalkohol+7 Ethylenoxid-tartrat-Natriumsalz (ca. 25 % Aktivsubstanz; CTFA-Bezeichnung: Sodium Laureth-7 Tartrate) (AUSICHEM) | |
| ⁴² Fettamin-Derivat mit Betainstruktur (ca. 30 % Aktivsubstanz; CTFA-Bezeichnung: Coco-Betaine) (HENKEL) | |
| ⁴³ Talgalkohol+60 Ethylenoxid-myristylether (CTFA-Bezeichnung: Talloweth60 Myristyl Glycol) (Akzo) | |

### 12) Shampoo

| | |
|---|---|
| Texapon^{R}N 70 | 19,4 |
| Akypo^{R}RLM 100 NV | 9,1 |
| Dehyton^{R}K | 6,7 |
| Plantaren^{R}-1200 | 4,0 |
| Merquat^{R}550⁴⁴ | 3,8 |
| D-Panthenol | 0,5 |
| Glucose | 1,0 |
| Benzoesäure | 0,5 |
| Polychol^{R}20 | 2,5 |
| Dehymuls^{R}SMO | 0,7 |
| Monomuls^{R}90 0 18 | 0,8 |
| Natriumchlorid | 0,7 |
| Wasser | ad 100 |
| Der pH-Wert des Shampoos betrug 5,0. | |

| | |
|---|---|
| ⁴⁴ Dimethyldiallylammoniumchlorid-Acrylamid-Copolymer (8 % Aktivsubstanz; CTFA-Bezeichnung: Polyquarternium 7) (MOBIL OIL) | |

### 13) Kurpackung

| | |
|---|---|
| Stenol^{R}1618 | 3,0 |
| Eumulgin^{R}B 1 | 0,5 |
| Eumulgin^{R}B 2 | 0,5 |
| Cutina^{R}CP | 1,0 |
| Eutanol^{R}G | 1,5 |
| Hostacerin^{R}PN 73⁴⁵ | 0,004 |
| Dow Corning^{R}929-Emulsion | 2,9 |
| Polychol^{R}5 | 1,5 |
| Dehymuls^{R}SSO | 1,0 |
| Monomuls^{R}90 0 18 | 1,0 |
| Wasser | ad 100 |

| | |
|---|---|
| ⁴⁵ Polyacrylsäure-Natriumsalz-Copolymer (HOECHST) | |

### 14) Färbecreme

| | |
|---|---|
| C₁₂₋₁₈-Fettalkohol | 1,2 |
| Lanette O | 4,0 |
| Eumulgin^{R}B 2 | 0,8 |
| Cutina^{R}KD 16⁴⁶ | 2,0 |
| Natriumsulfit | 0,5 |
| L(+)-Ascorbinsäure | 0,5 |
| Ammoniumsulfat | 0,5 |
| 1,2-Propylenglykol | 1,2 |
| Polymer JR^{R}400⁴⁷ | 0,3 |
| p-Aminophenol | 0,35 |
| p-Toluylendiamin | 0,85 |
| 2-Methylresorcin | 0,14 |
| 6-Methyl-m-aminophenol | 0,42 |
| Polychol^{R}5 | 0,5 |
| Dehymuls^{R}SMS | 0,3 |
| Monomuls^{R}90 0 18 | 0,3 |
| Ammoniak | 1,5 |
| Wasser | ad 100 |

| | |
|---|---|
| ⁴⁶ Fettsäuremono/diglycerid-Emulgator-Gemisch (CTFA-Bezeichnung: Tallow Glycerides (and) Glyceryl Stearate (and) Potassium Stearate) (HENKEL) | |
| ⁴⁷ quaternierte Hydroxyethylcellulose (Union Carbide) | |

Die Färbecreme hatte einen pH-Wert von 10,0. Sie bewirkte eine intensive rote Tönung des Haares.

### 15) Färbecreme

| | |
|---|---|
| Texapon^{R}N 25 | 25,0 |
| C₁₂₋₁₈-Fettalkohol | 2,0 |
| Hydrenol^{R}D⁴⁸ | 6,5 |
| Ammoniumsulfat | 1,1 |
| L(+)-Ascorbinsäure | 0,4 |
| Natriumsulfit | 0,5 |
| p-Aminophenol | 0,08 |
| p-Toluylendiamin | 0,6 |
| Resorcin | 0,11 |
| 4-Chlorresorcin | 0,23 |
| 6-Methyl-m-aminophenol | 0,06 |
| Ammoniak | 1,2 |
| Wasser | ad 100 |
| Der pH-Wert dieser Mischungskomponente betrug 10,0. | |

| | |
|---|---|
| ⁴⁸ C_{16/18}-Fettalkohol | |

### 15a) Entwickleremulsion für Farbcremes 14) und 15)

| | |
|---|---|
| Texapon^{R}N 25 | 2,1 |
| Wasserstoffperoxid (50%ig) | 12,0 |
| Turpinal^{R}SL⁴⁹ | 1,7 |
| Latekoll^{R}D⁵⁰ | 12,0 |
| Polychol^{R}20 | 0,9 |
| Dehymuls^{R}SMO | 0,5 |
| Monomuls^{R}90 0 18 | 0,7 |
| Wasser | ad 100 |

| | |
|---|---|
| ⁴⁹ 1-Hydroxyethan-1,1-diphosphonsäure (60 % Aktivsubstanz; CTFA-Bezeichnung: Etidronic Acid) (HENKEL) | |
| ⁵⁰ Acrylester-Methacrylsäure-Copolymer (25 % Aktivsubstanz) (BASF) | |

Die Färbecreme bewirkte eine braune Tönung des Haares.

### 16) Tönungsshampoo

| | |
|---|---|
| Texapon^{R}N 70 | 14,0 |
| Dehyton^{R}K | 10,0 |
| Akypo^{R}RLM 45 NV⁵¹ | 14,7 |
| Plantaren^{R}-1200 | 4,0 |
| Polymer P1, entsprechend DE 39 29 973 | 0,3 |
| Cremophor^{R}RH 40⁵² | 0,8 |
| Farbstoff C.I. 12 719 | 0,02 |
| Farbstoff C.I. 12 251 | 0,02 |
| Farbstoff C.I. 12 250 | 0,04 |
| Farbstoff C.I. 56 059 | 0,03 |
| PHB-Ester | 0,25 |
| Parfümöl | q.s. |
| Polychol^{R}20 | 1,6 |
| Dehymuls^{R}SML | 1,2 |
| Monomuls^{R}90 0 18 | 1,1 |
| Wasser | ad 100 |

| | |
|---|---|
| ⁵¹ Laurylalkohol+4,5 Ethylenoxid-essigsäure-Natriumsalz (20,4 % Aktivsubstanz) (CHEM-Y) | |
| ⁵² Rizinus-Öl, hydriert + 45 Ethylenoxid (CTFA-Bezeichnung: PEG-40 Hydrogenated Castor Oil) (BASF) | |

Beim Waschen der Haare mit diesem Tönungs-Shampoo erhalten diese einen glänzenden, hellblonden Farbton.

### 17) Cremedauerwelle

| Wellcreme | |
|---|---|
| Plantaren^{R}-800⁵³ | 5,0 |
| Thioglykolsäure | 8,0 |
| Turpinal^{R}SL | 0,5 |
| Ammoniak (25%ig) | 7,3 |
| Ammoniumcarbonat | 3,0 |
| Cetyl/Stearyl-Alkohol | 5,0 |
| Guerbet-Alkohol | 4,0 |
| Polychol^{R}5 | 3,0 |
| Dehymuls^{R}SMO | 2,0 |
| Monomuls^{R}90 0 18 | 1,0 |
| Parfümöl | q.s. |
| Wasser | ad 100 |
| Der pH-Wert der Wellcreme betrug 8,0. | |

| | |
|---|---|
| ⁵³ C₈-C₁₀-Alkylglucosid mit Oligomerisationsgrad 1,6 (ca. 60% Aktivsubstanz) (HENKEL) | |

| Fixierlösung | |
|---|---|
| Plantaren^{R}-800 | 5,0 |
| gehärtetes Rizinusöl | 2,0 |
| Kaliumbromat | 3,5 |
| Nitrilotriessigsäure | 0,3 |
| Zitronensäure | 0,2 |
| Merquat^{R}550 | 0,5 |
| Polychol^{R}20 | 1,5 |
| Dehymuls^{R}SMO | 1,0 |
| Monomuls^{R}90 0 18 | 1,0 |
| Parfümöl | q.s. |
| Wasser | ad 100 |
| Der pH-Wert der Fixierlösung betrug 3,8. | |

### 18) Flüssigdauerwelle

| Wellotion | |
|---|---|
| Plantaren^{R}-1200 | 3,0 |
| Thioglykolsäure | 8,0 |
| Dehyton^{R}K | 3,3 |
| Ammoniak (25%ig) | 6,4 |
| Ammoniumcarbonat | 3,0 |
| Lamequat^{R}L⁵⁴ | 2,9 |
| Nitrilotriessigsäure | 0,3 |
| Kerasol^{R} ⁵⁵ | 1,0 |
| Farbstoffe | q.s. |
| Parfümöl | q.s. |
| Wasser | ad 100 |

| | |
|---|---|
| ⁵⁴ kationisiertes Kollagenhydrolysat (ca. 35 % Aktivsubstanz; CTFA-Bezeichnung: Lauryldimonium Hydroxypropyl Hydrolyzed Collagen) (HENKEL) | |
| ⁵⁵ Proteinhydrolysat mit einer mittleren Molmasse von ca. 100.000 Dalton (13 % Aktivsubstanz) (CRODA) | |

| Fixierlösung | |
|---|---|
| Plantaren^{R}-1200 | 6,0 |
| Texapon^{R}N 25 | 5,4 |
| Kaliumbromat | 3,5 |
| Xanthan-Gummi | 0,3 |
| Zitronensäure | 0,1 |
| Dehyquart^{R}E⁵⁶ | 1,0 |
| Polychol^{R}20 | 0,8 |
| Dehymuls^{R}SSO | 0,8 |
| Monomuls^{R}90 0 18 | 1,0 |
| Parfümöl | q.s. |
| Wasser | ad 100 |

| | |
|---|---|
| ⁵⁶ N-(2-Hydroxyhexadecyl-1)-N,N-dimethyl-N(2-hydroxyethyl)ammoniumchlorid (28 % Aktivsubstanz) (HENKEL) | |

### 19) Geldauerwelle

| Wellgel | |
|---|---|
| Plantaren^{R}-1200 | 4,0 |
| Thioglykolsäure | 8,0 |
| Thiomilchsäure | 3,0 |
| Hydroxyethylcellulose | 0,7 |
| Turpinal^{R}SL | 0,5 |
| Ammoniak (25%ig) | 9,3 |
| Ammoniumcarbonat | 3,0 |
| Lamequat^{R}L | 2,9 |
| Kerasol^{R} | 0,5 |
| Parfümöl | q.s. |
| Wasser | ad 100 |

| Fixierlösung | |
|---|---|
| Plantaren^{R}-1200 | 6,0 |
| Wasserstoffperoxid (50%ig) | 12,0 |
| Texapon^{R}N 25 | 3,6 |
| Turpinal^{R}SL | 0,7 |
| Panthenol | 1,0 |
| Ajidew^{R}N 50⁵⁷ | 4,0 |
| Xanthan-Gummi | 0,1 |
| Polychol^{R}20 | 0,7 |
| Dehymuls^{R}SMO | 0,9 |
| Monomuls^{R}90 0 18 | 0,8 |
| Wasser | ad 100 |
| Die Fixierlösung hatte einen pH-Wert von 3,5. | |

| | |
|---|---|
| ⁵⁷ DL-2-Pyrrolidon-5-carbonsäure-Natriumsalz (ca. 50 % Aktivsubstanz; CTFA-Bezeichnung: Sodium PCA) (AJINOMOTO) | |

## Patentansprüche

1. Wäßrige Zubereitung zur Behandlung von Haaren enthaltend übliche kosmetische Bestandteile, dadurch gekennzeichnet, daß
- alkoxylierte Wollwachsalkohole (A),
- Ester des Glycerins mit gesättigten oder ungesättigten C₁₀-C₂₂-Fettsäuren (B) und
- Ester des Sorbitans mit gesättigten oder ungesättigten C₁₀-C₂₂-Fettsäuren (C) und/oder deren alkoxylierte Analoga enthalten sind.

2. Zubereitung nach Anspruch 1, dadurch gekennzeichnet, daß der alkoxylierte Wollwachsalkohol (A) 1 bis 50 Moleküle Ethylenoxid pro Alkoholmolekül enthält.

3. Zubereitung nach einem der Ansprüche 1 oder 2, dadurch gekennzeichnet, daß die Glycerinester (B) Monoester des Glycerins mit ungesättigten Fettsäuren sind.

4. Zubereitung nach einem der Ansprüche 1 bis 3, dadurch gekennzeichnet, daß die Sorbitanester (C) Mono-, Sesqui-, Di- oder Triester mit ungesättigten Fettsäuren sind.

5. Zubereitung nach einem der Ansprüche 1 bis 4, dadurch gekennzeichnet, daß sie
- 0,1 - 5 Gew.-% alkoxylierte Wollwachsalkohole (A)
- 0,1 - 3 Gew.-% Glycerinester (B) und
- 0,1 - 3 Gew.-% Sorbitanester (C) und/oder deren alkoxylierte Analoga enthält.

6. Zubereitung nach einem der Ansprüche 1 bis 5, dadurch gekennzeichnet, daß sie zusätzlich ein Konditionierungsmittel enthält.

7. Zubereitung nach Anspruch 6, dadurch gekennzeichnet, daß das Konditionierungsmittel ein Alkylamidoamin ist.

8. Zubereitung nach einem der Ansprüche 1 bis 7, dadurch gekennzeichnet, daß die Zubereitung einen pH-Wert zwischen 3,5 und 6, insbesondere zwischen 4,0 und 5,0 aufweist

9. Zubereitung nach einem der Ansprüche 1 bis 8, dadurch gekennzeichnet, daß zur Einstellung des pH-Wertes eine Genußsäure, insbesondere Zitronensäure oder Milchsäure, enthalten ist.

10. Zubereitung nach einem der Ansprüche 1 bis 9, dadurch gekennzeichnet, daß sie als Haarnachbehandlungsmittel formuliert ist.

11. Zubereitung nach einem der Ansprüche 1 bis 10, dadurch gekennzeichnet, daß sie als Shampoo formuliert ist.

12. Zubereitung nach einem der Ansprüche 1 bis 11, dadurch gekennzeichnet, daß sie als Haarfärbemittel formuliert ist.

13. Verfahren zur Behandlung von Haaren, dadurch gekennzeichnet, daß eine Zubereitung nach einem der Ansprüche 1 bis 12 auf das Haar aufgebracht wird.

14. Verfahren nach Anspruch 13, dadurch gekennzeichnet, daß das Haar anschließend mit Wasser oder einem im wesentlichen Wasser enthaltenden Mittel gespült wird.

## Claims

1. A water-based hair treatment preparation containing typical cosmetic constituents, characterized in that
- alkoxylated wool wax alcohols (A),
- esters of glycerol with saturated or unsaturated C₁₀₋₂₂ fatty acids (B) and
- esters of sorbitan with saturated or unsaturated C₁₀₋₂₂ fatty acids (C) and/or alkoxylated analogs thereof
are present.

2. A preparation as claimed in claim 1, characterized in that the alkoxylated wool wax alcohol (A) contains 1 to 50 molecules ethylene oxide per alcohol molecule.

3. A preparation as claimed in claim 1 or 2, characterized in that the glycerol esters (B) are monoesters of glycerol with unsaturated fatty acids.

4. A preparation as claimed in any of claims 1 to 3, characterized in that the sorbitan esters (C) are monoesters, sesquiesters, diesters or triesters with unsaturated fatty acids.

5. A preparation as claimed in any of claims 1 to 4, characterized in that it contains
- 0.1 to 5% by weight alkoxylated wool wax alcohols (A)
- 0.1 to 3% by weight glycerol esters (B) and
- 0.1 to 3% by weight sorbitan esters (C) and/or alkoxylated analogs thereof.

6. A preparation as claimed in any of claims 1 to 5, characterized in that it also contains a conditioning agent.

7. A preparation as claimed in claim 6, characterized in that the conditioning agent is an alkyl amidoamine.

8. A preparation as claimed in any of claims 1 to 7, characterized in that the preparation has a pH value of 3.5 to 6 and, more particularly, in the range from 4.0 to 5.0.

9. A preparation as claimed in any of claims 1 to 8, characterized in that an edible acid, particularly citric acid or lactic acid, is present for pH adjustment.

10. A preparation as claimed in any of claims 1 to 9, characterized in that it is formulated as a hair aftertreatment preparation.

11. A preparation as claimed in any of claims 1 to 10, characterized in that it is formulated as a shampoo.

12. A preparation as claimed in any of claims 1 to 11, characterized in that it is formulated as a hair dye.

13. A process for treating hair, characterized in that the preparation claimed in any of claims 1 to 12 is applied to the hair.

14. A process as claimed in claim 13, characterized in that the hair is subsequently rinsed with water or with a preparation essentially containing water.

## Revendications

1. Préparation aqueuse de soins capillaires contenant des composants cosmétiques usuels,
caractérisée en ce que
sont présents :
- des alcools de lanoline alcoxylés (A),
- des esters de glycérine et d'acides gras saturés ou insaturés en C₁₀-C₂₂ (B) et,
- des esters de sorbitane et d'acides gras saturés ou insaturés en C_{10-C}22 (C) et/ou leurs analogues alcoxylés.

2. Préparation selon la revendication 1,
caractérisée en ce que
l'alcool de lanoline alcoxylé (A) contient de 1 à 50 molécules d'oxyde d'éthylène par molécule d'alcool.

3. Préparation selon l'une des revendications 1 ou 2,
caractérisée en ce que
les esters de glycérine (B) sont des monoesters de la glycérine et d'acides gras insaturés.

4. Préparation selon l'une des revendications 1 à 3,
caractérisée en ce que
les esters de sorbitane (C)sont des mono-, sesqui-, di- ou triesters d'acides gras insaturés.

5. Préparation selon l'une des revendications 1 à 4,
caractérisée en ce que
elle contient :
- 0,1-5 % en poids d'alcools de lanoline alcoxylés (A),
- 0,1-3 % en poids d'esters de glycérine (B) et,
- 0,1-3 % en poids d'esters de sorbitane (C) et/ou de leurs analogues alcoxylés.

6. Préparation selon l'une des revendications 1 à 5,
caractérisée en ce que
elle contient en plus un agent de conditionnement.

7. Préparation selon la revendication 6,
caractérisée en ce que
l'agent de conditionnement est une alkylamidoamine.

8. Préparation selon l'une des revendications 1 à 7,
caractérisée en ce que
la préparation présente un pH compris entre 3,5 et 6, notamment entre 4,0 et 5,0.

9. Préparation selon l'une des revendications 1 à 8,
caractérisée en ce que
un acide alimentaire est présent pour régler le pH notamment l'acide citrique ou l'acide lactique.

10. Préparation selon l'une des revendications 1 à 9,
caractérisée en ce que
elle est formulée comme agent de post-traitement capillaire.

11. Préparation selon l'une des revendications 1 à 10,
caractérisée en ce que
elle est formulée comme shampooing.

12. Préparation selon l'une des revendications 1 à 11,
caractérisée en ce que
elle est formulée comme teinture capillaire.

13. Procédé de soins des cheveux,
caractérisé en ce que
on applique une préparation selon l'une des revendications 1 à 12 sur les cheveux.

14. Procédé selon la revendication 13,
caractérisé en ce que
ensuite on rince les cheveux avec de l'eau ou avec un moyen contenant essentiellement de l'eau.
